Europäisches Patentamt

European Patent Office

Office européen des brevets

(11) Veröffentlichungsnummer: **0 446 874 A2**

# EUROPÄISCHE PATENTANMELDUNG

(21) Anmeldenummer: **91103768.7**

(22) Anmeldetag: **12.03.91**

(51) Int. Cl.5: **A61B 5/05**

(30) Priorität: **14.03.90 DE 9003076 U**

(43) Veröffentlichungstag der Anmeldung:
**18.09.91 Patentblatt 91/38**

(84) Benannte Vertragsstaaten:
**AT DE FR IT**

(71) Anmelder: **WERNER EIDAM
MEDIZIN-TECHNOLOGIE GMBH
Robert-Bosch-Strasse 23
W-6307 Linden(DE)**

(72) Erfinder: **Eidam, Werner**

**Robert-Bosch-Strasse 23
W-6307 Linden(DE)**
Erfinder: **Heidelbach, Wolfgang, Dipl.-Ing.
Breiter Weg 101
W-6307 Linden(DE)**
Erfinder: **Mayr, Harald
Wissmarer Weg 33
W-6300 Giessen(DE)**

(74) Vertreter: **Olbricht, Karl Heinrich, Dipl.-Phys.
Am Weinberg 15
W-3556 Niederweimar(DE)**

(54) **Medizinisches Messgerät.**

(57) Zur elektrophysikalischen Zustandserfassung insbesondere in der Mundhöhle dient ein medizinisches Meßgerät (10) mit zwei beweglichen Aufsetzelektroden (20) und mit einer an der Geräte-Frontplatte (14) angeordneten visuellen Anzeigeeinrichtung (28), die mit einer Eingabe-Tastatur (26) sowie mit einem Druckwerk (30) gekoppelt und zur Darstellung eines zeitlichen Meßwert-Verlaufs ausgebildet ist, z.B. mit einem rechteckigen LCD-Sichtfeld. Das Druckwerk (30) kann an der Oberseite des Gehäuses (12) eingebaut sein, namentlich mit nach oben austretendem Druckstreifen (32). Zumindest eine Aufsetzelektrode (20) kann einen Meßgriffel (22) mit einem durch Fingerkontakt betätigbaren Sensorschalter (24) aufweisen oder bilden. Meß- und Schalteinrichtungen (44, 62; 26, 46) ist eine Steuereinrichtung zugeordnet, namentlich ein Mikroprozessor (48) mit Programmspeicher (50) sowie Taktgeber und Rückstellvorrichtung. Die Stromversorgung (40) kann eine zeitgesteuerte Sparschaltung sowie eine Ladeanzeige aufweisen und netzunabhängig sein.

Fig. 1

Die Erfindung betrifft ein medizinisches Meßgerät gemäß dem Oberbegriff von Anspruch 1.

Derartige Meßgeräte werden in der Mundheilkunde eingesetzt, wo es eine Rolle spielt, welche physiologischen Vorgänge deswegen auftreten, weil im Mund metallische Werkstoffe wie Silberamalgam, Gold u.dgl. vorhanden sind. In Verbindung mit dem Speichel entstehen dadurch elektrische Potentialdifferenzen, die örtliche Reizwirkungen auf die Schleimhaut ausüben und auch Fernwirkungen wie Allergien im Körper erzeugen können.

Man hat Meßanordnungen zur Feststellung der so bedingten Leerlaufspannungen und Kurzschlußströme entwickelt, wie etwa von D. G. Lucas in Dtsch. zahnärztl. Zeitschrift 28 (1973) 394ff. berichtet. Als störend erwies sich, daß die Meßwerte wegen unterschiedlicher Geräte-Innenwiderstände nicht vergleichbar waren. Auch blieben Meßwert-Veränderungen unberücksichtigt; vielmehr versuchte man eine Beurteilung anhand statischer Meßergebnisse. So wurden als obere Grenzwerte für die Mundbatterie-Strombildung, bei deren Überschreitung eine Gebißsanierung zu empfehlen sei, geräteabhängig eine Spannung von 80 mV oder 100 mV oder 130 mV und ein Kurzschlußstrom von z.B. 3 $\mu$A oder 5 $\mu$A oder 10 $\mu$A angegeben.

Eine Untersuchung von F. Kramer/H. Peesel in Zahnärztl. Praxis 28 (1977) 332ff. bezog den Stromabfall an einem gegebenen Lastwiderstand und den Energieinhalt nach einer Meßdauer von z.B. 1,5 s ein, so daß methodisch verläßlichere Aussagen erzielbar wurden. Im Ergebnis wurde aber weiterhin an bestimmten Zahlenwerten festgehalten, die teils auf der Verwendung eines bestimmten Geräts, teils auf persönlicher Erfahrung beruhen.

Ein als kombiniertes Potentialgerät bezeichnetes, handliches Meßgerät verfügt über einen elektronischen Speicher, um bei Einzel- und Serienmessungen von Spannung und Strom den jeweils entstandenen Maximalstrom zu speichern und durch Tastendruck erneut anzeigen zu können. Hierbei ist allerdings keine Energiemessung vorgesehen.

Ein wichtiges Ziel der Erfindung besteht darin, die Beschränkungen des Standes der Technik zu überwinden und mit möglichst einfachen, wirtschaftlichen Mitteln ein Gerät der letztgenannten Art so zu verbessern, daß eine umfassendere Ermittlung des elektrophysikalischen Zustands im Mund rasch möglich ist. Ferner soll die Zuverlässigkeit der Diagnose gesteigert und die Beurteilung der Schädlichkeit einer Mundbatterie weiter objektiviert werden können.

Ein Hauptmerkmal der Erfindung ist im kennzeichnenden Teil von Anspruch 1 angegeben. Ausgestaltungen sind Gegenstand der Ansprüche 2 bis 12.

Die Erfindung sieht vor, daß die visuelle Anzeigeeinrichtung des Geräts mit einer Eingabe-Tastatur sowie mit einem Druckwerk gekoppelt ist. Dies geht über die herkömmliche Technik hinaus und erlaubt es, nicht nur die an der Anzeigeeinrichtung visuell erfaßbaren Meß- und Speicherwerte nach Bedarf abzurufen und zu vergleichen, sondern außerdem zur Dokumentation einzeln oder fortlaufend auszudrucken.

In der Ausgestaltung nach Anspruch 2 ist vorgesehen, daß die visuelle Anzeigeeinrichtung zur Darstellung eines zeitlichen Meßwert-Verlaufs ausgebildet ist. Sie kann laut Anspruch 3 ein insbesondere rechteckiges LCD-Sichtfeld aufweisen. Im praktischen Einsatz wird damit - für Arzt und Patient gut sichtbar - beispielsweise der Leistungsverlauf während der gewählten Meßdauer graphisch wiedergegeben. Ein solches anschauliches Diagramm ist schon qualitativ aussagekräftig; die Meßdaten der Anzeige gestatten außerdem die quantitative Auswertung.

Vorteilhaft ist gemäß Anspruch 4 das Druckwerk in das Gehäuse eingebaut, so daß eine kompakte Baueinheit geschaffen ist. Nach Anspruch 5 ist das Druckwerk an der Oberseite des Gehäuses angeordnet, namentlich mit nach oben austretendem Druckstreifen wodurch bequeme Bedien- und Ablesbarkeit gewährleistet ist.

Eine weitere, sehr zweckmäßige Ausgestaltung besteht laut Anspruch 6 darin, daß die Eingabe-Tastatur in das Gehäuse insbesondere frontseitig eingebaut ist und/oder daß Anschlüsse für eine zusätzliche Eingabe-Tastatur vorgesehen sind. Dies erweitert die Möglichkeiten des Geräts, das bei bequemer Handhabung schon mit der eingebauten Eingabe-Tastatur z.B. ein Abrufen gespeicherter Meßwert-Verläufe erlaubt. Die Anschaltung einer zusätzlichen Tastatur gestattet die Aufnahme und das Ausdrucken weiterer Daten.

Bei einem Gerät mit frontseitig am Gehäuse angeordneten Anschlüssen für die Aufsetzelektroden sieht die Erfindung gemäß Anspruch 7 ferner vor, daß zumindest eine Aufsetzelektrode einen Meßgriffel mit einem Auslöseschalter, vorzugsweise einem durch Fingerkontakt betätigbaren Sensorschalter, aufweist oder bildet. Das unterstützt die bequeme Bedienung, indem ein Meßzyklus lediglich durch die Handhabung der Elektroden ausgelöst wird.

In sehr zweckmäßiger Ausgestaltung sieht die Erfindung gemäß Anspruch 8 vor, daß den Meß- und Schalteinrichtungen eine Steuereinrichtung zugeordnet ist, namentlich ein Mikroprozessor mit Programmspeicher. Die Steuereinrichtung kann ferner laut Anspruch 9 einen Taktgeber sowie eine Rückstellvorrichtung aufweisen. Sowohl die Abwicklung und Auswertung der Messungen als auch die Möglichkeiten der Geräteverwendung werden dadurch noch erheblich vielfältiger.

Für den Betrieb des Gerätes ist es sehr vorteilhaft, wenn nach Anspruch 10 die visuelle Anzeigeeinrichtung zur Darstellung der Betriebsbereitschaft ausgebildet ist, insbesondere mit einer Ladeanzeige der Stromversorgung. Letztere kann gemäß Anspruch 11 mit einer zeitgesteuerten Sparschaltung versehen sein. Der Benutzer erkennt mithin ohne weiteres, ob bzw. wann die Betriebsbereitschaft des Gerätes vorhanden ist. Ist sie vorübergehend nicht gegeben, so erscheint eine entsprechende Anzeige auf dem Sichtfeld. Die freie Verwendung des Meßgeräts wird stark erleichtert, indem laut Anspruch 12 eine netzunabhängige Stromversorgung eingebaut ist, bevorzugt mit einem Blei-Gel-Akkumulator, dessen Ladezustand von dem Mikroprozessor fortlaufend überwacht wird.

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnung. Darin zeigen:

Fig. 1 eine schematisierte Vorder-Schrägansicht eines Meßgerätes,

Fig. 2 ein Blockschaltbild der Meß- und Schalteinrichtungen eines Meßgerätes entsprechend Fig. 1 und

Fig. 3 sieben beispielhafte Meßdiagramme.

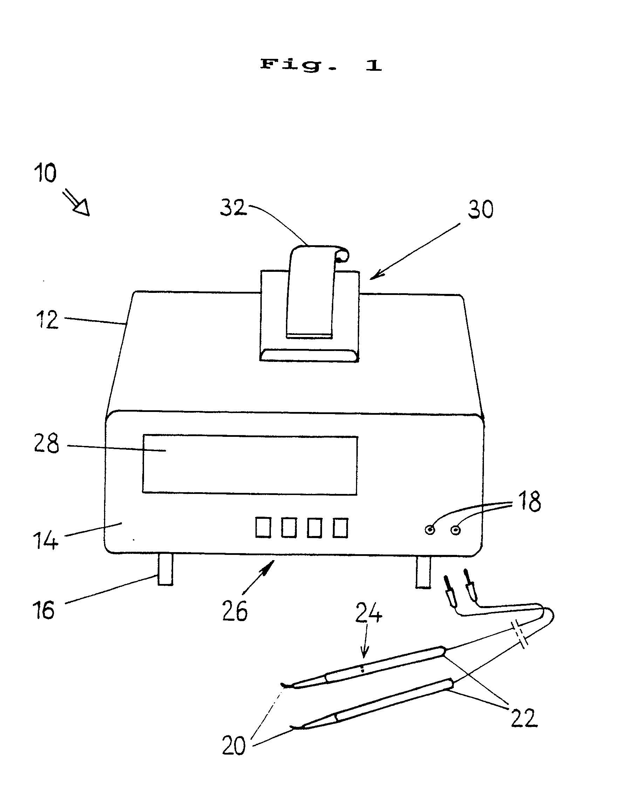

Das in Fig. 1 schematisch veranschaulichte Meßgerät ist allgemein mit 10 bezeichnet. Es hat ein Gehäuse 12 mit einer Frontplatte 14 sowie Schwenkfüße 16, die es gestatten, das Gehäuse 12 vorn anzuheben und mithin den Blickwinkel zur Frontplatte 14 zu verändern.

An der Frontplatte 14 befinden sich Anschlüsse 18 für Aufsetzelektroden 20, die jeweils einen Meßgriffel 22 haben. Die eine Aufsetzelektrode 20 hat einen Sensorschalter 24, der durch Fingerberührung die Meßbereitschaft des Geräts 10 aktiviert.

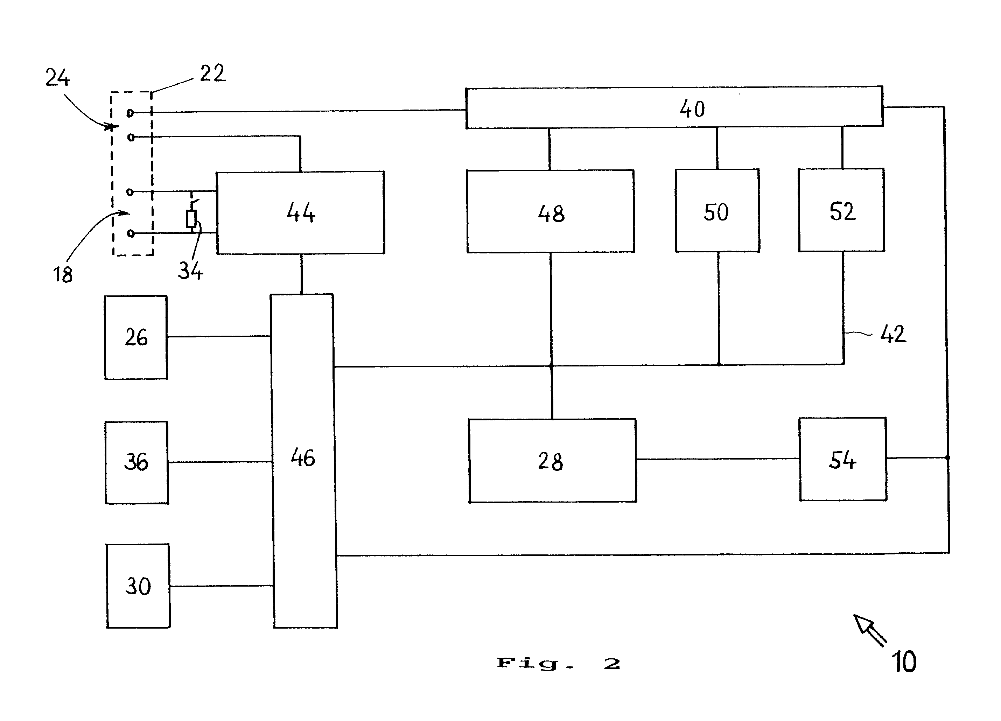

Ferner befindet sich an der Frontplatte 14 eine Eingabe-Tastatur 26 und ein LCD-Display 28. Letzteres ist zur Darstellung eines zeitlichen Meßwert-Verlaufs ausgebildet, insbesondere der zwischen den Aufsetzelektroden 20 während einer gewählten Zeitdauer gemessenen, veränderlichen Leistung. Außerdem können am LCD-Display 28 Meßwerte wie eine elektrische Ladung, eine elektrische Spannung und sonstige Meßdaten angezeigt werden. Ein Teil des Sichtfeldes ermöglicht es ferner, den Ladezustand einer Stromversorgung 40 (Fig. 2) visuell anzuzeigen.

An der Oberseite des Gehäuses 12 ist ein Druckwerk 30 so angeordnet, daß ein Druckstreifen 32 nach oben und hinten austreten kann. Ist der Druckstreifen 32 verbraucht, so kann eine neue Papierrolle bequem von oben in das Druckwerk 30 eingesetzt werden.

Wie aus Fig. 2 hervorgeht, dient die Stromversorgung 40 zur Speisung einer Reihe von Einheiten. Zu diesen gehört ein Meßverstärker 44, dem der Sensorschalter 24 zugeordnet ist. An einen Schaltblock 46, der als Parallel-Input-Output ausgebildet sein kann, sind die Eingabe-Tastatur 26 und das Druckwerk 30 angeschlossen. Bei Bedarf kann eine separate zweite Tastatur 36 und/oder ein weiteres Druckwerk angeschlossen werden.

Die Anschlüsse 18 der Aufsetzelektroden 20 führen zum Eingang des Meßverstärkers 44, der zweckmäßig mit einem Analog/Digital-Wandler ohne oder mit Multiplexer versehen ist. Der Ausgang führt zu einer Sammelschiene oder einem Bus 42, an den ein Mikroprozessor 48 mit zugehörigem Programmspeicher 50 sowie ein Meßspeicher 52 angeschlossen sind. Mit dem Bus 42 ist ferner der Schaltblock 46 sowie die Anzeigeeinrichtung bzw. das LCD-Display 28 verbunden, welches über einen Inverter 54 von der Stromversorgung 40 gespeist wird.

Der Mikroprozessor 48 überwacht laufend den Ladezustand der Stromversorgung 40 und schaltet bei zu geringer Spannung eines (nicht gezeichneten) Akkumulators auf Zwangsladen um; im Normalbetrieb kann die Umschaltung zeitgesteuert vor sich gehen, beispielsweise etwa 20 min nach dem letzten Bedienungsvorgang.

Ein (nicht gezeichneter) Betriebsschalter kann an der Geräterückseite angeordnet sein. Nach seiner Betätigung zeigt das LCD-Display 28 den Ladezustand der Stromversorgung 40. Der Meßablauf wird durch Fingerkontakt am Sensorschalter 24 ausgelöst.

Ein typischer Meßablauf sieht zunächst die Ermittlung der Leerlaufspannung an einem Innenwiderstand von 1 MOhm vor. Dann erfolgt die Zuschaltung eines Lastwiderstandes 34 von beispielsweise 10 kOhm, bevorzugt während einer Meßdauer von 1,5 s. Nach dem Sampling-Verfahren wird außer der Spannung am Lastwiderstand 34 noch der momentane Strom und die jeweilige Leistung ermittelt. Die am Lastwiderstand 34 abfallende Spannung steuert eine Stromquelle, deren Ausgang zur Aufladung eines (nicht dargestellten) Kondensators benutzt wird. Die am Ende der Meßdauer vorhandene Ladung des Kondensators wird im LCD-Display 28 angezeigt. Sodann wird der Kondensator durch elektronischen Kurzschluß entladen.

Der Meßspeicher 52 kann beispielsweise achtzig Meßvorgänge aufnehmen; er kann dann durch Tastendruck gelöscht werden.

Im Programmspeicher 50 sind typische Diagramme abgelegt, die - ebenso wie Meßdiagramme - durch Tastendruck nacheinander betrachtet werden können. Vorzugsweise durch Pfeile gekennzeichnete Tasten erlauben ein Vor- und Zurückblättern in den verschiedenen Diagrammen. Diese können durch Betätigung einer Taste der Eingabe-Tastatur 26 zur Dokumentation ausgedruckt werden. Weitere Tasten oder Tastenkombinationen erlauben es, zusätzliche Daten auszudrukken, insbesondere Angaben zur Person sowie Meßwerte von Energie, Spannung und/oder Strom. Diese Daten lassen sich vorzugsweise über eine serielle Schnittstelle auch an einen externen Drucker bzw. Plotter oder an einen angeschlossenen Computer ausgeben. Eine weitere Anzeigeeinrichtung, z.B. mit größerem und eventuell farbigem Sichtfeld, kann ebenfalls angeschlossen werden, desgleichen eine zweite Tastatur 36.

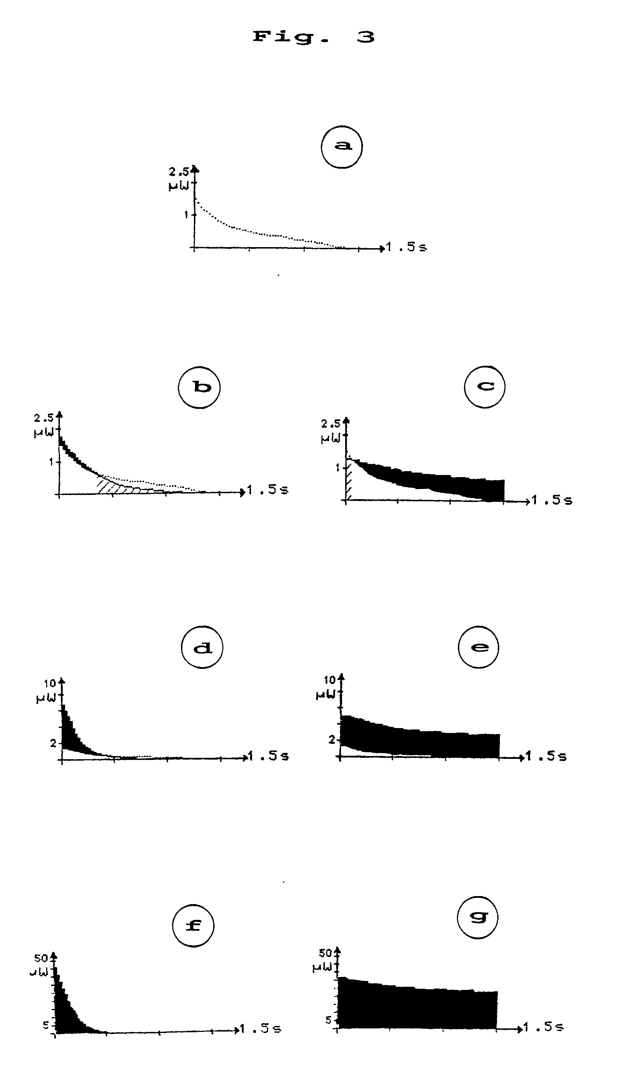

Fig. 3 zeigt Beispiele von Standard-Diagrammen, die mit den Buchstaben a bis g gekennzeichnet sind. Das obere Diagramm a veranschaulicht eine Art Normkurve, wie sie den bisherigen Erkenntnissen zugrunde gelegt werden kann. Diagramm b zeigt den typischen Meßverlauf, wenn die Aufsetzelektroden 20 an eine schwache, ladungsarme Mundbatterie angelegt werden und eine schnelle Entladung stattfindet. Eine gleichfalls schwache, jedoch ladungsreiche Batterie führt bei langsamer Entladung zum Diagramm c, dessen dunklerer Flächenteil mehr Energieinhalt bedeutet.

In entsprechender Gegenüberstellung veranschaulicht das Diagramm d den Leistungsverlauf bei einer mittleren, ladungsarmen Mundbatterie mit schneller Entladung, während im Diagramm e eine ebenfalls mittlere, aber ladungsreiche Mundbatterie sich nur langsam entlädt und im dunklen Bereich einen noch größeren Energieinhalt erkennen läßt.

Das Diagramm f zeigt eine sich schnell entladende, starke, aber ladungsarme Mundbatterie. Dem ist das Diagramm g einer ebenfalls starken, jedoch ladungsreichen Mundbatterie mit langsamer Entladung gegenübergestellt, wobei die dunkle Fläche einen hohen Energieinhalt bereits qualitativ anzeigt.

Durch Vergleich mit solchen Musterdiagrammen kann jeder einzelne Meßverlauf sinnvoll eingeordnet und der Mundhöhlen-Zustand zuverlässig diagnostiziert werden. Die Erfindung ist dabei nicht auf die obengenannte Anzahl gespeicherter Diagramme beschränkt.

Vielmehr kann durch geeignete andere Speichereinrichtungen auch eine weit größere Anzahl von Meßabläufen gespeichert werden.

Auch die Reihenfolge der Meßabläufe läßt sich auf einfache Weise verändern, beispielsweise mittels der Eingabe-Tastatur 26 oder einer äußeren zweiten Tastatur 36.

Während herkömmlich der Beurteilung meist nur wenige Zahlenwerte und mehr oder weniger willkürliche Toleranzgrenzen zugrunde gelegt wurden, gestattet es die Erfindung erstmals, solchen Zahlenwerten bestimmte Inhalte nicht nur zuzuordnen, sondern daraus quantitative Schlüsse zu ziehen. Beispielsweise müßte eine nach dem Stand der Technik ermittelte verhältnismäßig hohe Spannung nicht unbedingt eine Schädlichkeit der Mundbatterie bedeuten, weil eine schnelle Entladung zu raschem Zusammenbruch der Spannung führen konnte. Die erfindungsgemäß dargebotenen Schaubilder geben jedoch auch den zeitlichen Verlauf wieder, so daß eine tatsächlich schädliche Mundbatterie nun sicher erkannt werden kann. Dabei ist wichtig, daß mit dem erfindungsgemäßen Gerät außer Strom, Spannung und Leistung zwischen den Aufsetzelektroden 20 auch die jeweils transportierte Ladung ermittelt werden kann. Sie ist eine wertvolle Meßgröße, welche die Zuverlässigkeit der Diagnose ebenso unterstützt wie der graphisch unmittelbar anschauliche Leistungsverlauf, der auf dem LCD-Display bzw. einer sonstigen visuellen Anzeigeeinrichtung 26 dargeboten wird.

Aus dem Vorstehenden wird deutlich, daß erfindungsgemäß zur elektrophysikalischen Zustandserfassung insbesondere in der Mundhöhle ein medizinisches Meßgerät 10 mit zwei beweglichen Aufsetzelektroden 20 und mit einer an der Geräte-Frontplatte 14 angeordneten visuellen Anzeigeeinrichtung 28 vorgesehen ist, die mit einer Eingabe-Tastatur 26 sowie mit einem Druckwerk 30 gekoppelt und zur Darstellung eines zeitlichen Meßwert-Verlaufs ausgebildet ist, z.B. mit einem rechteckigen LCD-Sichtfeld. Das Druckwerk 30 kann an der Oberseite des Gehäuses 12 eingebaut sein, namentlich mit nach oben austretendem Druckstreifen 32. Zumindest eine Aufsetzelektrode 20 kann einen Meßgriffel 22 mit einem durch Fingerkontakt betätigbaren Sensorschalter 24 aufweisen oder bilden. Meß- und Schalteinrichtungen 44, 62; 26, 46 ist eine Steuereinrichtung zugeordnet, namentlich ein Mikroprozessor 48 mit Programmspeicher 50 sowie Taktgeber und Rückstellvorrichtung. Die Stromversorgung 40 kann eine zeitgesteuerte Sparschaltung sowie eine Ladeanzeige aufweisen und netzunabhängig sein.

Sämtliche aus den Ansprüchen, der Beschreibung und der Zeichnung hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

Legende PA 579 EP

| | |
|---|---|
| 10 | Gerät |
| 12 | Gehäuse |
| 14 | Frontplatte |
| 16 | Schwenkfüße |
| 18 | Anschlüsse |
| 20 | Aufsetzelektroden |
| 22 | Meßgriffel |
| 24 | Sensorschalter |

26 Eingabe-Tastatur
28 LCD-Display
30 Druckwerk
32 Druckstreifen
34 Lastwiderstand
36 zweite Tastatur
40 Stromversorgung
42 Bus
44 Meßverstärker
46 Schaltblock
48 Mikroprozessor
50 Programmspeicher
52 Meßspeicher
54 Inverter

## Patentansprüche

1. Medizinisches Meßgerät (10), insbesondere zur elektrophysikalischen Zustandserfassung in der Mundhöhle, mit einem eine Frontplatte (14) aufweisenden Gehäuse (12), mit zwei beweglich angeschlossenen oder anschließbaren, griffelartig isolierten Aufsetzelektroden (20), mit Einrichtungen (44, 52) zum Messen und Speichern von Spannung und Strom zwischen den Aufsetzelektroden während einer gewählten Meßdauer, mit Schalteinrichtungen (26, 46) und mit einer visuellen Anzeigeeinrichtung (28) an der Geräte-Frontplatte (14), dadurch **gekennzeichnet,** daß die visuelle Anzeigeeinrichtung (28) mit einer Eingabe-Tastatur (26) sowie mit einem Druckwerk (30) gekoppelt ist.

2. Gerät nach Anspruch 1, dadurch **gekennzeichnet,** daß die visuelle Anzeigeeinrichtung (28) zur Darstellung eines zeitlichen Meßwert-Verlaufs ausgebildet ist.

3. Gerät nach Anspruch 1 oder 2, dadurch **gekennzeichnet,** daß die visuelle Anzeigeeinrichtung (28) ein insbesondere rechteckiges LCD-Sichtfeld aufweist.

4. Gerät nach einem der Ansprüche 1 bis 3, dadurch **gekennzeichnet,** daß das Druckwerk (30) in das Gehäuse (12) eingebaut ist und/oder daß Anschlüsse für ein separates Druckwerk vorgesehen sind.

5. Gerät nach Anspruch 4, dadurch **gekennzeichnet,** daß das Druckwerk (30) an der Oberseite des Gehäuses (12) angeordnet ist, namentlich mit nach oben austretendem Druckstreifen (32).

6. Gerät nach einem der Ansprüche 1 bis 5, dadurch **gekennzeichnet,** daß die Eingabe-Tastatur (26) in das Gehäuse (12) insbesondere frontseitig eingebaut ist und/oder daß Anschlüsse für eine zusätzliche Eingabe-Tastatur (46) vorgesehen sind.

7. Gerät nach einem der Ansprüche 1 bis 6, mit frontseitig am Gehäuse (12) angeordneten Anschlüssen (18) für die Aufsetzelektroden (20), dadurch **gekennzeichnet,** daß zumindest eine Aufsetzelektrode (20) einen Meßgriffel (22) mit einem· Auslöseschalter (24), vorzugsweise einem durch Fingerkontakt betätigbaren Sensorschalter, aufweist oder bildet.

8. Gerät nach wenigstens einem der Ansprüche 1 bis 7, dadurch **gekennzeichnet,** daß den Meß- und Schalteinrichtungen (44, 52; 26, 46) eine Steuereinrichtung zugeordnet ist, namentlich ein Mikroprozessor (48) mit Programmspeicher (50).

9. Gerät nach Anspruch 8, dadurch **gekennzeichnet,** daß die Steuereinrichtung (48) einen Taktgeber sowie eine Rückstellvorrichtung aufweist.

10. Gerät nach wenigstens einem der Ansprüche 1 bis 9, dadurch **gekennzeichnet,** daß die visuelle Anzeigeeinrichtung (28) zur Darstellung der Betriebsbereitschaft ausgebildet ist, insbesondere mit einer Ladeanzeige der Stromversorgung (40).

11. Gerät nach Anspruch 10, dadurch **gekennzeichnet,** daß die Stromversorgung (40) eine zeitgesteuerte Sparschaltung aufweist.

12. Gerät wenigstens nach Anspruch 10, dadurch **gekennzeichnet,** daß eine netzunabhängige Stromversorgung (40) eingebaut ist, bevorzugt mit einem Blei-Gel-Akkumulator, dessen Ladezustand von dem Mikroprozessor (48) fortlaufend überwacht wird.

Fig. 1

Fig - 2

Fig. 3